# FASCICULE DE BREVET EUROPEEN

(11) **EP 0 938 366 B1**
(45) Date de publication et mention de la délivrance du brevet: **20.02.2002**
(21) Numéro de dépôt: 97943941.1
(22) Date de dépôt: 03.10.1997
(51) Int. Cl.: B01D 61/44

(54) **PROCEDE D'EXTRACTION DE COMPOSES AMINES D'UN MILIEU LIQUIDE**
VERFAHREN ZUM EXTRAHIEREN VON AMINVERBINDUNGEN AUS EINEM FLÜSSIGEN MEDIUM
METHOD FOR EXTRACTING AMINE COMPOUNDS FROM A LIQUID MEDIUM

(30) Priorité: 04.10.1996 FR 9612327
(43) Date de publication de la demande: 01.09.1999
(73) Titulaire: Rhodia, 92408 Courbevoie Cedex (FR)
(72) Inventeur: CANIVENC, Edith, F-69008 Lyon (FR); HORBEZ, Dominique, F-95130 Franconville (FR)
(74) Mandataire: Esson, Jean-Pierre
(86) Numéro de dépôt international: FR9701760
(87) Numéro de publication internationale: WO9815341

(56) Documents cités:
- WO-A-91/02584
- WO-A-93/25299
- WO-A-93/25513
- WO-A-93/25514
- DE-A- 3 627 280
- FR-A- 1 223 635
- FR-A- 1 267 568
- US-A- 4 238 306
- KEN-ICHI KIKUCHI ET AL: "SEPARATION OF AMINO ACIDS BY ELECTRODIALYSIS WITH ION-EXCHANGE MEMBRANES" JOURNAL OF CHEMICAL ENGINEERING OF JAPAN, vol. 28, no. 1, 1 février 1995, pages 103-109, XP000502994

## Description

La présente invention concerne un procédé d'extraction par électrodialyse selon la revendication 1.

Les matières synthétiques et parmi ceux-ci les polymères contenant des fonctions amides tels que les polyamides et plus particulièrement le polyamide 6.6 ou le polyamide 6, sont de plus en plus utilisés pour la réalisation d'articles variés tels que fibres textiles, fils industriels techniques, filaments ou articles moulés comme pièces pour l'électricité, l'électronique ou l'automobile. Ces matériaux synthétiques sont généralement utilisés sous forme de compositions comprenant en plus du matériau synthétique, différents additifs pour, par exemple, augmenter leur stabilité thermique, ou aux rayonnements, améliorer leurs propriétés mécaniques, électriques ou électrostatiques, des colorants, des pigments. Ces additifs sont de nature très variée et peuvent être aussi bien des composés organiques que minéraux.

En outre, notamment pour la réalisation d'articles moulés, les compositions comprennent des charges souvent minérales telles que les fibres de verre, du talc, de l'argile etc...

Les matériaux ainsi produits peuvent être détruits après usage. Un des moyens de destruction classique est l'incinération qui permet de récupérer de l'énergie. Toutefois, il est également proposé de recycler ces matériaux.

En effet, une des préoccupations actuelles réside dans le recyclage des produits obtenus à partir de ressources naturelles pour d'une part ralentir l'épuisement de ces ressources et d'autre part diminuer le volume des rejets dans la nature.

Ainsi, des procédés de dépolymérisation des polyamides ont été proposés. Un des procédés consiste à réaliser une hydrolyse en milieu acide, neutre ou basique du polyamide pour obtenir soit des oligomères de degré de polymérisation faible, soit des sels. Le procédé d'hydrolyse s'applique notamment pour la dépolymérisation des polyamides type PA 6 c'est-à-dire obtenus par homopolycondensation d'un aminoacide ou d'un lactame.

Dans le cas des polyamides type PA 6.6, c'est-à-dire obtenus par polymérisation entre une diamine et un diacide, l'hydrolyse doit être conduite en milieu alcalin pour obtenir une dépolymérisation totale. Dans le cas d'une hydrolyse en milieu neutre, le milieu d'hydrolysat contient un mélange d'oligomères de degré de polymérisation différent et compris généralement entre 2 et 40, et plus précisément entre 2 et 20.

L'hydrolyse en milieu alcalin présente un inconvénient majeur car il est nécessaire pour récupérer le monomère acide, par exemple l'acide adipique, de faire réagir l'adipate formé avec un acide, par exemple l'acide nitrique. Ce procédé est donc consommateur de produit (acide) et générateur d'un effluent (sels d'alcalin). Pour remédier à ce problème un procédé électrolytique de transformation du sel adipate en acide adipique a été proposé dans les demandes de brevets internationales WO 93/25299, WO 93/25514 et WO 93/25513.

Il a également été proposé, dans les demandes françaises FR 95/08916 et FR 95/08917 un procédé permettant d'hydrolyser thermiquement les polyamides en milieu neutre, puis transformer les oligomères ainsi obtenus en monomères amines, acides, aminoacides ou en sels d'amine par hydrolyse enzymatique. Ces composés dissous dans le milieu d'hydrolyse peuvent être extraits et séparés par différents procédés tels que cristallisation, précipitation ou distillation. Toutefois, ces procédés classiques d'extraction et de purification ont des rendements faibles et ne sont pas très sélectifs.

Un des objets de la présente invention est de proposer un procédé qui permet de séparer sélectivement les différents composés produits et notamment les composés comprenant au moins une fonction protonable amine.

A cet effet, l'invention propose un procédé selon la revendication 1.

Ainsi, la cellule élémentaire d'électrodialyse permettant de mettre en oeuvre l'invention comprend une membrane cationique disposée entre deux électrodes.

Selon une autre caractéristique de l'invention, le pH de la solution est déterminé et ajusté pour correspondre au point isoélectrique ou au pKa du composé à extraire par passage à travers la membrane cationique, et plus avantageusement au pKa de l'amine à séparer.

On parlera de point isoélectrique quand le composé à extraire comprend plusieurs fonctions ionisables respectivement positivement et négativement telles que des fonctions amines et des fonctions acides.

Ainsi, le procédé de l'invention permet de séparer différents composés contenus dans un milieu liquide par extraction sélective d'un des composés, ou par réalisation d'une séparation en plusieurs étapes avec extraction d'un composé à chaque étape avec modification du pH du milieu liquide entre chaque étape pour l'adapter au point isoélectrique ou pKa du composé à extraire dans ladite étape.

Dans un mode de réalisation préféré de l'invention, notamment quand le milieu liquide comprend des monomères produits par la dépolymérisation de polyamides, la cellule d'électrodialyse élémentaire comprend avantageusement une membrane cationique et une membrane anionique

Ainsi cette cellule comprend un compartiment de dilution D dans lequel est alimenté le milieu liquide à traiter, et deux compartiments de concentration (C⁺, C⁻) dans lesquels les composés extraits du milieu liquide et traversant sous l'effet du courant électrique les membranes respectivement cationique et anionique, se concentrent. Quand des cellules sont montées en série électrique, le compartiment C⁺ d'une cellule formera également le compartiment C⁻ de la cellule adjacente dans la série, et inversement pour le compartiment C⁻. En conséquence, pour plus de clarté nous nommerons C tous les compartiments de concentration, sans distinction de signes.

En effet, le procédé de l'invention trouve une application préférentielle dans la séparation et la récupération des monomères produits par un procédé de dépolymérisation de polyamides.

Ainsi, l'hydrolyse en milieu neutre de polyamide conduit à une solution aqueuse de monomères diamines, diacides et/ou aminoacides selon la nature des polyamides traités, ainsi que des oligomères de degré de polymérisation compris par exemple entre 2 et 40.

Le traitement d'une telle solution par le procédé de l'invention permet d'extraire et de séparer les monomères diamines et diacides, des oligomères et aminoacides.

Toutefois, pour améliorer le rendement de dépolymérisation et transformer les oligomères en monomères, la solution aqueuse décrite ci-dessus est soumise à une hydrolyse enzymatique.

Des exemples d'hydrolyse enzymatique des oligomères de polyamides et plus particulièrement d'oligomères de polyadipamide d'hexaméthylène (PA 6.6) sont décrits dans les demandes de brevet français n° 95 08916 et n° 95 08917 dont les textes sont incorporés à la présente.

Brièvement, les procédés décrits consistent à réaliser une hydrolyse thermique en milieu neutre de polyamides, puis à traiter cet hydrolysat avec des enzymes appelées "amidases". Ces enzymes sont, par exemple, celle (PAM I) exprimée par la souche E.COli contenant le plasmide PXL 2564 déposée à la collection Nationale de Culture et Microorganismes sous le n° I 1495, le 29 novembre 1994, l'enzyme produite par la souche nyl-B décrite dans Kinoshita et al. (Eur. J. Biochem 116, 547-551, 1981), l'amidase (PAM II) exprimée par la souche Comonas Acidovorans déposée à la Collection Nationale des Cultures de Microorganismes sous le n° I 1522 le 4 janvier 1995.

La solution récupérée après hydrolyse enzymatique contient uniquement des sels d'amines et/ou les monomères diamines et diacides, lactames ou aminoacides selon la nature des polyamides utilisés.

Le procédé de l'invention permet d'extraire les monomères diamine d'un tel milieu, et dans le cas d'une électrodialyse avec membranes cationique et anionique, d'extraire d'une part les diamines protonées à travers la membrane cationique et d'autre part les monomères diacides à travers la membrane anionique, dans les compartiments C de concentration respectifs.

Les composés tels que les lactames, aminoacides, oligomères ne seront pas extraits sous l'effet du courant électrique, et resteront dans le compartiment D de la dilution.

Selon un mode de réalisation préféré de l'invention, le procédé est mis en oeuvre dans une cellule comprenant au moins deux cellules élémentaires d'électrodialyse montées en série électrique.

Ainsi, le compartiment C de concentration d'une cellule élémentaire sera également le compartiment C de concentration de la cellule adjacente. En conséquence, la diamine extraite à travers la membrane cationique et le diacide extrait à travers la membrane anionique de la cellule adjacente seront présents dans le même milieu et formeront un sel d'amine.

Une autre application du procédé de l'invention est un procédé de purification ou d'extraction d'un sel d'amine d'un milieu liquide.

En effet, par ajustement du pH du milieu liquide les fonctions amines du sel d'amine seront protonées, et sous l'effet du courant le composé à fonction amine traversera la membrane cationique tandis que le composé à fonction acide migrera vers le compartiment de concentration C⁻ à travers la membrane anionique. Ces deux composés extraits du milieu de départ se recombineront dans le compartiment C commun de concentration de deux cellules adjacentes pour reformer le sel d'amine, sous une forme purifiée ou séparée des autres composés éventuellement contenus dans le milieu liquide de départ.

Le procédé de l'invention est préférentiellement utilisé pour la séparation et l'extraction des monomères diamines contenus dans le milieu d'hydrolyse de polyamides. Pour cette application le pH du milieu liquide est avantageusement compris entre 6 et 9, de préférence entre 7 et 8. Le pH du milieu liquide peut être ajusté par tout moyen connu tel que par addition de composés basiques ou acides.

Ce procédé trouve notamment une application dans la séparation, la récupération et purification des monomères produits par hydrolyse thermique complétée par une hydrolyse enzymatique de polyamides contenant notamment un polyadipamide d'hexaméthylène. Ce polyamide peut être en mélange avec d'autres polyamides tels que du polycaprolactame ou être sous forme de copolyamide.

Le procédé de l'invention permet, dans cette application, de séparer l'hexaméthylène diamine et l'acide adipique des autres monomères provenant par exemple du polycaprolactame comme l'acide aminocaproïque ou le caprolactame, ainsi que des oligomères non complètement hydrolysés ou de sous-produits de l'hydrolyse.

Les concentrations en composés à extraire ou séparer, notamment en sels d'amine ou monomères amines et acides dans le milieu liquide ne sont pas critiques. Toutefois, ces concentrations pourront être avantageusement comprises entre 0,2 mol/l et 2 mol/l, de préférence 0,2 mol/l à 1 mol/l.

Pour la mise en oeuvre du procédé, les appareils d'électrodialyse classiques sont convenables.

Ainsi, un appareil d'électrodialyse convenable pour la mise en oeuvre du procédé est constitué par différents compartiments délimités respectivement par des membranes cationiques et des membranes anioniques. Ces compartiments se divisent en compartiments de dilution D qui s'appauvrissent en composé à séparer, c'est-à-dire en diacides et diamines ou sels d'amine correspondant dans le procédé de l'invention, et en compartiments de concentration C qui à l'inverse s'enrichissent en composés à séparer.

En effet, sous l'action du champ électrique les amines protonées de la solution à traiter migrent vers la cathode en sortant du compartiment D où ils se trouvent, à travers une membrane échangeuse de cations (membrane cationique). Lorsqu'ils sont passés dans le compartiment C suivant, ils ne peuvent pas le quitter en raison de la présence de la membrane échangeuse d'anions (membrane anionique) suivante. Simultanément les composés acides migrent vers l'anode en traversant une membrane anionique et passent dans un compartiment C⁻ adjacent, qu'ils ne peuvent ensuite pas quitter en raison de la présence de la membrane cationique suivante.

Deux compartiments C et D adjacents forment une cellule d'électrodialyse. Un électrodialyseur comporte un empilement de plusieurs cellules. Ce nombre de cellules par électrodialyseur est de manière générale aussi élevé que possible. Par exemple, ce nombre peut varier avantageusement entre 5 et 500 cellules.

En pratique, les membranes anioniques et cationiques sont disposées alternativement dans un système de type filtre-presse.

Les membranes homopolaires utilisées dans le procédé de l'invention se divisent en deux grandes familles, selon leur mode de fabrication.

Ce sont tout d'abord les membranes hétérogènes, préparées à partir de résines échangeuses d'ions, mélangées à un liant tel que polychlorure de vinyle, polyéthylène ou autre. L'ensemble ainsi formé peut enduire une trame comme par exemple un tissu de polyester ou de polyacrylonitrile.

Ce sont également les membranes homogènes, obtenues par introduction d'un groupement fonctionnel sur un support inerte, par greffage chimique ou radiochimique. La méthode chimique, la plus utilisée, consiste généralement à fonctionnaliser un latex d'un polymère comportant des noyaux aromatiques, tel que styrène/divinylbenzène ou styrène/butadiène. Le latex ainsi fonctionnalisé sert ensuite à enduire une trame comme pour les membranes hétérogènes. La méthode radiochimique comporte généralement le greffage, sous l'influence d'un rayonnement, d'un composé aromatique, tel que le styrène, sur un support inerte comme une feuille de polyéthylène ou de polytétrafluoroéthylène. Le noyau aromatique est ensuite fonctionnalisé comme dans la méthode chimique.

Les membranes échangeuses de cations (membranes cationiques) comportent des groupements acides forts, le plus souvent des groupements sulfonates, ou des groupements acides faibles, souvent des groupements carboxylates. Plus rarement les groupements acides peuvent être des groupements PO₃²⁻, HPO₂⁻, AsO₃²⁻, SeO₃⁻.

Les membranes échangeuses d'anions (membranes anioniques) comportent des groupement basiques forts, le plus souvent des groupements ammonium quaternaire, ou des groupements basiques faibles, le plus souvent des groupements amines. Plus rarement les groupements basiques peuvent être des groupements phosphonium quaternaire ou des groupements sulfonium.

Dans le présent procédé, les membranes cationiques comportent de préférence des groupements acides forts et parmi ceux-ci préférentiellement des groupements sulfonates et les membranes anioniques comportent de préférence des groupements basiques forts et parmi ceux-ci préférentiellement des groupements ammonium quaternaire.

Selon un autre mode de réalisation de l'invention, l'électrodialyse est réalisée en disposant dans chaque compartiment commun de concentration C une membrane bipolaire.

La membrane bipolaire est un assemblage de deux membranes, l'une cationique, l'autre anionique. Lorsque la membrane est soumise à un champ électrique suffisant, l'eau de solvatation à l'interface de la membrane se dissocie en ions H⁺ et OH⁻, qui migrent respectivement vers la cathode en traversant la face cationique et vers l'anode en traversant la face anionique. Comme membranes bipolaires, on peut citer à titre d'exemple les membranes commercialisées par les sociétés Aqualytics, Tokuyama Soda, FuMaTech.

Ainsi, dans ce mode de réalisation, l'appareil d'électrodialyse est constitué par différents compartiments délimités respectivement par des membranes cationiques, des membranes bipolaires et des membranes anioniques. Ces compartiments se divisent en compartiment de dilution D qui s'appauvrissent en composés à séparer, en compartiment acide C⁻ et amine C⁺ où se concentrent respectivement l'acide et l'amine extraits du compartiment D.

En effet, sous l'action du champ électrique, l'amine protonée migre vers la cathode en sortant du compartiment D où elle se trouve, à travers une membrane échangeuse de cations (membrane cationique). Lorsqu'elle est passée dans le compartiment C⁺ suivant, l'amine est déprotonée par l'apport des ions OH⁻ provenant de la face anionique de la membrane bipolaire, au sein de laquelle s'effectue la dissociation de l'eau sous l'effet du champ électrique. L'amine est ainsi régénérée.

Simultanément, les ions carboxylate (adipate) migrent vers l'anode en sortant du compartiment D où ils se trouvent, à travers une membrane échangeuse d'anions (membrane anionique). Lorsqu'ils sont passés dans le compartiment (C⁻) suivant, ils sont protonés par l'apport des ions H⁺ provenant de la face cationique de la membrane bipolaire. Les trois compartiments C⁺, C⁻, D adjacents forment une cellule d'électrodialyse. Un électrodialyseur comporte un empilement de plusieurs cellules. Par exemple, ce nombre peut varier avantageusement entre 5 et 300 cellules.

Outre les membranes, l'électrodialyseur comporte bien entendu une cathode et une anode. L'anode est constituée en des matériaux classiquement utilisés en électrodialyse, par exemple en graphite ou en nickel, en titane revêtu par des métaux précieux ou des oxydes de métaux précieux, notamment le titane platiné. La cathode est également constituée en des matériaux classiquement utilisés en électrodialyse, par exemple en graphite, en acier inoxydable, en nickel.

L'électrodialyseur est alimenté avec la solution aqueuse à traiter. Il est également nécessaire de faire circuler à l'anode une solution d'un anolyte et à la cathode une solution d'un catholyte. Ces solutions constituent souvent une solution unique d'électrolyte. Dans le présent procédé, un circuit unique d'électrolyte convient bien. Le rôle de la solution d'électrolyte est d'assurer une conductivité suffisante. De préférence, cette conductivité sera égale ou supérieure à 20 millisiemens par centimètre (mS/cm), sans que cette limite inférieure soit à considérer comme critique pour la mise en oeuvre du présent procédé.

L'électrolyte mis en oeuvre est un composé ionisable tel qu'un sel, un acide ou une base. L'électrolyte est de préférence choisi parmi les composés non électroactifs. Ainsi, par exemple, il est préférable de ne pas utiliser industriellement des chlorures qui généreraient du chlore à l'anode.

On peut citer comme exemples d'électrolytes, des sels neutres comme les sulfates, des acides comme l'acide sulfurique, des bases comme la soude.

La tension ou la densité de courant appliquée à l'électrodialyseur sans membrane bipolaire doit être de nature à éviter la polarisation du système, c'est-à-dire une dissociation de l'eau sous l'effet d'un champ électrique trop intense. A titre d'exemple, les densités de courant utilisées sont d l'ordre de 0,15 kA/m², cette valeur ne doit pas être considérée comme une valeur maximale. Dans le cas d'une électrodialyse avec membrane bipolaire, la densité de courant appliquée est généralement plus élevée pour polariser ladite membrane. Ainsi, les densités de courant appliquées sont généralement comprise entre 0,2 et 1,5 kA / m², de préférence entre 0,2 et 0,5 kA / m². On préfère des cellules comprenant des compartiments d'épaisseur comprise entre 0,5 mm et 2 mm et de préférence entre 0,6 mm et 1,5 mm.

La température à laquelle est mis en oeuvre le procédé de l'invention se situe dans un domaine compatible avec la stabilité des membranes. En effet, si en principe les températures élevées sont favorables, en accroissant la mobilité électrolytique et en réduisant la viscosité de la solution à traiter, l'augmentation de la température diminue la durée de vie des membranes. On opérera donc de préférence à une température inférieure ou égale à 70°C et plus particulièrement entre 20°C et 60°C.

L'électrodialyseur peut fonctionner de différentes façons. Il peut tout d'abord fonctionner en continu (fonctionnement en passage direct), la solution à traiter traversant en continu l'empilement ; plusieurs étages sont alors disposés en série hydraulique si le taux de traitement à obtenir l'exige. Il peut aussi fonctionner en discontinu (fonctionnement en recirculation), la solution à traiter recirculant sur une cuve jusqu'à ce que le taux de traitement souhaité soit obtenu. Enfin il peut fonctionner en passage direct avec une recirculation partielle.

Par ailleurs, pour obtenir un bon fonctionnement de l'électrodialyse, la conductivité électrique des solutions contenues dans les compartiments de concentration C⁺, C⁻ doit être suffisante. Ainsi, dans le mode de réalisation avec membrane bipolaire, la solution de diamine présente dans les compartiments C⁺ peut présenter une conductivité trop faible pour permettre un fonctionnement correct de la cellule d'électrodialyse. Dans ce cas, la conductivité électrique pourra être augmentée par addition d'une faible quantité d'électrolyte. Le choix de l'électrolyte doit être réalisé en fonction de sa non-réactivité avec l'amine et de la possibilité de le séparer facilement de l'amine pour récupérer celle-ci à l'état de pureté maximum.

Cette augmentation de la conductivité est également valable pour la solution du compartiment C⁻ contenant le composé acide.

En outre, le volume de la solution circulant ou contenue dans les compartiments C⁻, notamment dans le cas de l'électrodialyse avec membrane bipolaire doit être suffisant pour avoir une concentration en composés acides à la fin de l'électrodialyse ou en sortie de compartiment inférieure à la concentration de saturation dudit composé acide, à la température de fonctionnement de la cellule. Ainsi, toute précipitation de composés acides est évitée.

Par ailleurs, il est préférable de filtrer le milieu liquide à traiter avant son alimentation dans la cellule d'électrodialyse pour ainsi éviter de colmater les membranes.

D'autres buts, avantages et détails de l'invention apparaîtront plus clairement au vu des exemples donnés uniquement à titre indicatif et ne présentent aucun caractère limitatif.

### Exemple 1

L'électrodialyseur est un empilement de 10 cellules de 2 dm² de surface active, chaque cellule étant composée de 2 compartiments :
- un compartiment D limité côté anode par une membrane échangeuse d'anions commercialisée par la société Tokuyama Soda sous le nom commercial Neosepta AMX et du côté cathode par une membrane échangeuse de cations commercialisée par la même société sous le nom Neosepta CMX,
- un compartiment C limité du côté de l'anode par la membrane cationique ci-dessus et du côté de la cathode par la membrane anionique de la cellule adjacente.

L'électrolyte circulant au niveau des électrodes est une solution de Na₂SO₄ présentant une conductivité électrique de 20 mS / cm.

La solution alimentant les compartiments C est une solution contenant 5 g /l de NaCl.

La température de mise en oeuvre du procédé est la température ambiante, de l'ordre de 22°C.

La tension imposée entre les électrodes est de 18 volt.

La solution alimentée dans le compartiment D a la composition suivante :
- acide adipique : 7,25 % en poids (0,5 mole /l)
- hexaméthylène diamine (HMD) : 5,99 % en poids (0,52 mole /l)
- acide amino-6 caproïque : 7,30 % en poids
- caprolactame : 5,65 % en poids
- pH : 8,4

Cette composition correspond à celle obtenue par hydrolyse totale d'un mélange de PA 6.6 et PA 6.

Cette solution est soumise à une électrodialyse pendant 30 minutes.

La solution du compartiment C analysée après arrêt de l'électrodialyse a la composition suivante (à l'exception du NaCl):
- acide adipique : 1,18 %
- HMD: 0,67%
- acide amino-6 caproïque : < 0,04 %
- caprolactame : < 25 ppm

Ces résultats montrent clairement l'effet de séparation de l'acide adipique et de l'hexaméthylène diamine, c'est-à-dire du sel adipate d'hexaméthylène diamine par rapport au caprolactame et à l'acide amino-6 caproïque.

Le rendement faradique par rapport à HMD est de l'ordre de 84 % tandis qu'il est de l'ordre de 100 % par rapport à l'acide adipique.

### Exemple 2

Cet exemple a été réalisé dans les mêmes conditions que l'exemple 1 mais avec une solution de départ présentant la composition suivante :
- acide adipique : 3,63 % en poids (0,25 mole /l)
- hexaméthylène diamine : 2,99 % en poids (0,26 mole / l)
- acide amino-6 caproïque : 3,65 % en poids
- caprolactame : 2,83 % en poids

L'électrodialyse a été poursuivie jusqu'à épuisement en HMD et acide adipique de la solution contenue dans le compartiment D.

La solution récupérée dans le compartiment C a la composition suivante :
- acide adipique : 3,30 %
- HMD : 2,36 %
- acide amino-6 caproïque : < 0,02 %
- caprolactame : < 0,1 %

On obtient ainsi un sel d'adipate d'hexaméthylène diamine contenant moins de 3500 ppm d'acide amino-6-caproïque et 1,7 % de caprolactame.

### Exemple 3

Cet exemple a été réalisé dans une cellule d'électrodialyse comprenant deux compartiments séparés par une membrane cationique commercialisée par la société E.I. Du Pont de Nemours sous le nom commercial NAFION ® 324.

Le compartiment anodique contient initialement 300 ml d'une solution résultant de l'hydrolyse à la soude de polyamide 6.6 et ayant la composition pondérale suivante :
- adipate de sodium : 139 g / l (0,73 mole / l)
- hexaméthylènediamine : 83 g / l (0,73 mole / l)
- le pH de la solution est initialement de 12,5.

Le compartiment cathodique contient initialement 250 cm³ d'une solution de soude à 5 % en poids.

L'électrodialyse est réalisée à 65°C avec une intensité de courant de 20 A / dm².

La concentration en HMD dans le compartiment cathodique est mesurée en fonction du temps, ainsi que le pH de la solution du compartiment D anodique.

Les résultats sont indiqués dans le tableau ci-dessous.

| temps (mn) | pH anolyte | [HMD] catholyte |
|---|---|---|
| 0 | 12,5 | non détecté |
| 240 | 11,5 | non détecté |
| 340 | 11,0 | non détecté |
| 360 | 10,5 | 0,19 mole / l |
| 380 | 9,0 | 0,24 mole / l |
| 400 | 6,5 | 0,29 mole / l |

Après 400 Minutes d'électrolyse, 40 % de l'HMD présente initialement dans l'anolyte sont passés dans le compartiment cathodique.

### Exemple 4

L'électrodialyseur utilisé est constitué d'un empilement de 5 cellules de 2 dm² de surface active, composées chacune d'elles de 3 compartiments désignés comme suit :
- un compartiment (D) : limité côté anode par une membrane échangeuse d'anions commercialisée par la société Tokuyama Soda sous le nom commercial Neosepta AHA-2, et du côté cathode par une membrane échangeuse de cations du nom commercial Neosepta CMB,
- un compartiment (C⁻) : limité du côté cathode par la membrane anionique, et du côté anode par la face cationique d'une membrane bipolaire de nom commercial Aqualytics,
- un compartiment (C⁺) : limité du côté anode par la membrane cationique, et du côté cathode par la face anionique de la membrane bipolaire.

L'anode est constituée de titane platiné. La cathode est en acier inoxydable.

L'électrolyte est constitué par une solution aqueuse de sulfate de sodium à 160 g / l. Le débit de circulation aux électrodes est de 2 x 100 l / h. Le volume est de 5 l.

Le compartiment C⁺ est initialement rempli de 5,2 litres d'une solution d'hydroxyde de sodium à 5 g / l.

Le compartiment C⁻ est initialement rempli de 7,5 litres d'une solution d'acide nitrique à 1 %.

Le compartiment D est initialement rempli de 4 kg d'une solution ayant la composition pondérale suivante :
- adipate d'hexaméthylènediamine : 12,5 %
- caprolactame : 5,5 %.
- acide amino-6 caproïque : 7 %
- eau: 75 %

Le débit de recirculation des solutions est fixé à 80 l / h pour les circuits des compartiments C⁺ et C⁻, et à 60 l / h pour le circuit du compartiment D.

L'électrodialyse est effectuée en mode discontinu (fonctionnement en recirculation), à une température moyenne de 42°C. L'intensité est imposée à 5 A, soit une densité de courant de 0,25 kA / m².

Après 248 minutes de fonctionnement, la conductivité du compartiment D est passée de 22,9 à 1,8 mS / cm.

Le volume final du compartiment C⁺ est de 5,86 litres. La concentration en hexaméthylène diamine est de 0,254 mole / l, soit un taux d'extraction ou récupération de 78 %.

Le volume final du compartiment C⁻ est de 7,92 litres. La concentration en acide adipique est de 0,183 mole / l, soit un taux d'extraction ou récupération de 76 %.

Le rendement faradique est compris entre 75 et 77 %.

## Revendications

1. Procédé d'extraction en milieu liquide de composés comprenant au moins deux fonctions protonables amines, **caractérisé en ce que** le milieu liquide comprend des monomères diamines obtenus par dépolymérisation de polyamides et **en ce que** le procédé consiste à protoner les fonctions amines des diamines par réglage du pH du milieu, et à séparer les composés par passage à travers une membrane cationique sous l'effet du courant électrique.

2. Procédé selon la revendication 1, **caractérisé en ce que** les composés comprenant au moins deux fonctions amines protonables sont un sel d'amine.

3. Procédé selon la revendication 2, **caractérisé en ce que** le sel d'amine est choisi dans le groupe comprenant les adipates d'amine, les phtalates d'amine.

4. Procédé selon l'une des revendications 1 à 3, **caractérisé en ce que** le composé amine est l'hexaméthylène diamine.

5. Procédé selon l'une des revendications 1 à 4, **caractérisé en ce que** l'électrodialyse comprend au moins une membrane anionique et au moins une membrane cationique formant une cellule élémentaire d'électrodialyse, avec un compartiment (D) de dilution et des compartiments (C) de concentration.

6. Procédé selon la revendication 5, **caractérisé en ce que** l'électrodialyse comprend au moins deux cellules élémentaires montées en série électrique.

7. Procédé selon la revendication 6, **caractérisé en ce qu'**il consiste à protoner les fonctions amines du sel d'amine contenu dans une solution, à alimenter ladite solution dans le compartiment (D), et à faire passer, sous l'effet du courant électrique, dans les compartiments de concentration commun (C), à travers les membranes cationiques et anioniques respectivement l'amine protonée et l'anion du sel d'amine, et à reformer dans lesdits compartiments (C) le sel d'amine extrait.

8. Procédé selon l'une des revendications 6 ou 7, **caractérisé en ce que** l'électrodialyse comprend une membrane bipolaire disposée dans le compartiment (C) situé entre deux cellules élémentaires d'électrodialyse montées en série électrique.

9. Procédé selon l'une des revendications précédentes, **caractérisé en ce que** le milieu liquide est obtenu par hydrolyse de polyamides.

10. Procédé selon la revendication 9, **caractérisé en ce que** le milieu liquide est obtenu par hydrolyse thermique suivi d'une hydrolyse enzymatique de polyamides.

11. Procédé selon l'une des revendications 1 à 10, **caractérisé en ce que** les polyamides sont choisis dans le groupe comprenant le polyamide 6.6, le polyamide 4.6, les polyamides semi-aromatiques du type polyphtalamide, les mélanges de ceux-ci et leurs copolyamides.

12. Procédé selon l'une des revendications précédentes, **caractérisé en ce que** la concentration molaire des composés à séparer dans le milieu liquide est comprise entre 0,2 mole/l et 2 mole/l.

13. Procédé de production de monomère de polyamide, **caractérisé en ce qu'**il consiste à hydrolyser les polyamides pour obtenir un milieu liquide contenant les monomères des polyamides de départ, et à séparer les monomères diacides, diamines et aminoacides ou lactames par électrodialyse du milieu liquide selon l'une des revendications 6 à 12.

14. Procédé selon la revendication 13, **caractérisé en ce que** l'hydrolyse est une hydrolyse thermique en milieu neutre suivie d'une hydrolyse enzymatique.

## Patentansprüche

1. Verfahren zur Extraktion im flüssigen Medium von Verbindungen, die mindestens zwei protonisierbare Aminfunktionen umfassen, **dadurch gekennzeichnet, daß** das flüssige Medium Diamin-Monomere umfaßt, erhalten durch Depolymerisation von Polyamiden, und dadurch, daß das Verfahren darin besteht, die Aminfunktionen der Diamine durch Regulierung des pH-Wertes des Mediums zu protonisieren und die Verbindungen mittels Passage durch eine kationische Membran unter der Wirkung des elektrischen Stromes zu trennen.

2. Verfahren nach Anspruch 1, **dadurch gekennzeichnet, daß** die Verbindungen, die mindestens zwei protonisierbare Aminfunktionen umfassen, Aminsalze sind.

3. Verfahren nach Anspruch 2, **dadurch gekennzeichnet, daß** das Aminsalz aus der Gruppe gewählt wird, die Aminadipate und Aminphthalate umfaßt.

4. Verfahren nach einem der Ansprüche 1 bis 3, **dadurch gekennzeichnet, daß** die Aminverbindung Hexamethylendiamin ist.

5. Verfahren nach einem der Ansprüche 1 bis 4, **dadurch gekennzeichnet, daß** die Elektrodialyse mindestens eine anionische Membran und mindestens eine kationische Membran umfaßt, die eine elementare Elektrodialysezelle bilden, mit einem Abteil (D) der Verdünnung und mit Abteilen (C) der Konzentration.

6. Verfahren nach Anspruch 5, **dadurch gekennzeichnet, daß** die Elektrodialyse mindestens zwei elementare Zellen umfaßt, die in elektrischer Reihe angeordnet sind.

7. Verfahren nach Anspruch 6, **dadurch gekennzeichnet, daß** es darin besteht, die Aminfunktionen des in einer Lösung enthaltenen Aminsalzes zu protonisieren, die genannte Lösung in das Abteil (D) einzuspeisen und das protonisierte Amin und das Anion des Aminsalzes durch jeweils kationische und anionische Membranen unter der Wirkung des elektrischen Stromes in die gemeinsamen Abteile (C) der Konzentration passieren zu lassen und in den Abteilungen (C) das extrahierte Aminsalz wieder neu zu bilden.

8. Verfahren nach einem der Ansprüche 6 oder 7, **dadurch gekennzeichnet, daß** die Elektrodialyse eine in dem Abteil (C) angeordnete bipolare Membran umfaßt, die sich zwischen zwei elementaren Elektrodialysezellen befindet, die in elektrischer Reihe angeordnet sind.

9. Verfahren nach einem der vorstehenden Ansprüche, **dadurch gekennzeichnet, daß** das flüssige Medium durch Hydrolyse von Polyamiden erhalten wird.

10. Verfahren nach Anspruch 9, **dadurch gekennzeichnet, daß** das flüssige Medium durch thermische Hydrolyse von Polyamiden, gefolgt von einer enzymatischen Hydrolyse, erhalten wird.

11. Verfahren nach einem der Ansprüche 1 bis 10, **dadurch gekennzeichnet, daß** die Polyamide aus der Gruppe gewählt werden, die Polyamid 6.6, Polyamid 4.6, die halbaromatischen Polyamide vom Typ Polyphthalamid, deren Mischungen und ihre Copolyamide umfaßt.

12. Verfahren nach einem der vorstehenden Ansprüche, **dadurch gekennzeichnet, daß** die molare Konzentration der in dem flüssigen Medium zu trennenden Verbindungen zwischen 0,2 mol/l und 2 mol/l beträgt.

13. Verfahren zur Herstellung von Polyamid-Monomeren, **dadurch gekennzeichnet, daß** es darin besteht, die Polyamide zu hydrolysieren, um ein flüssiges Medium zu erhalten, das die Monomeren der Ausgangs-Polyamide enthält, und die monomeren Disäuren, Diamine und Aminosäuren oder Lactame durch Elektrodialyse des flüssigen Mediums nach einem der Ansprüche 6 bis 12 zu trennen.

14. Verfahren nach Anspruch 13, **dadurch gekennzeichnet, daß** die Hydrolyse eine thermische Hydrolyse im neutralen Medium ist, gefolgt von einer enzymatischen Hydrolyse.

## Claims

1. Process for extracting, in liquid medium, compounds comprising at least two protonatable amine functions, **characterized in that** the liquid medium comprises diamine monomers obtained by depolymerization of polyamides, and **in that** the process consists in protonating the amine functions of the diamines by adjusting the pH of the medium and in separating the compounds by passing them through a cationic membrane under the effect of the electric field.

2. Process according to Claim 1, **characterized in that** the compounds comprising at least two protonatable amine functions are an amine salt.

3. Prccess according to Claim 2, **characterized in that** the amine salt is chosen from the group comprising amine adipates and amine phthalates.

4. Process according to one of Claims 1 to 3, **characterized in that** the amine compound is hexamethylenediamine.

5. Process according to one of Claims 1 to 4, **characterized in that** the electrodialysis comprises at least one anionic membrane and at least one cationic membrane forming an elemental electrodialysis cell, with a dilution compartment (D) and concentration compartments (C) .

6. Process according to Claim 5, **characterized in that** the electrodialysis comprises at least two elemental cells mounted in electrical series.

7. Process according to Claim 6, **characterized in that** it consists in protonating the amine functions of the amine salt contained in a solution, in feeding the said solution into the compartment (D), and in passing, under the effect of the electric current, into the common concentration compartments (C), across the cationic and anionic membranes respectively, the protonated amine and the anion of the amine salt, and in reforming the extracted amine salt in the said compartments (C).

8. Process according to either of Claims 6 and 7, **characterized in that** the electrodialysis comprises a bipolar membrane arranged in the compartment (C) located between two elemental electrodialysis cells mounted in electrical series.

9. Process according to one of the preceding claims, **characterized in that** the liquid medium is obtained by hydrolysis of polyamides.

10. Process according to Claim 9, **characterized in that** the liquid medium is obtained by thermal hydrolysis followed by an enzymatic hydrolysis of polyamides.

11. Process according to one of Claims 1 to 10, **characterized in that** the polyamides are chosen from the group comprising polyamide 6.6, polyamide 4.6, semi-aromatic polyamides of the polyphthalamide type, mixtures thereof and copolyamides thereof.

12. Process according to one of the preceding claims, **characterized in that** the molar concentration of the compounds to be separated in the liquid medium is between 0.2 mol/l and 2 mol/l.

13. Process for producing polyamide monomer, **characterized in that** it consists in hydrolysing the polyamides in order to obtain a liquid medium containing the monomers of the starting polyamides, and in separating the diacid, diamine and amino acid or lactam monomers by electrodialysis of the liquid medium according to one of Claims 6 to 12.

14. Process according to Claim 13, **characterized in that** the hydrolysis is a thermal hydrolysis in neutral medium followed by an enzymatic hydrolysis.
